# EUROPÄISCHE PATENTSCHRIFT

(11) **EP 1 041 948 B1**
(45) Veröffentlichungstag und Bekanntmachung des Hinweises auf die Patenterteilung: **16.04.2003**
(21) Anmeldenummer: 98960858.3
(22) Anmeldetag: 22.12.1998
(51) Int. Cl.: A61F 6/00

(54) **APPLIKATORRING FÜR KONDOME**
APPLICATOR RING FOR CONDOMS
ANNEAU APPLICATEUR POUR PRESERVATIFS

(30) Priorität: 22.12.1997 AT 216897
(43) Veröffentlichungstag der Anmeldung: 11.10.2000
(73) Patentinhaber: Liehs, Reinhard, 5020 Salzburg (AT)
(72) Erfinder: Liehs, Reinhard, 5020 Salzburg (AT)
(74) Vertreter: Babeluk, Michael, Dipl.-Ing. Mag.
(86) Internationale Anmeldenummer: AT9800318
(87) Internationale Veröffentlichungsnummer: WO99032058

(56) Entgegenhaltungen:
- DE-C- 823 318
- FR-A- 2 727 858

## Beschreibung

Die vorliegende Erfindung betrifft einen Applikatorring für Kondome mit einem Ring, der das Kondom aufgespannt hält, gemäß dem Oberbegriff von Anspruch 1.
Bisher werden Kondome in aufgerolltem Zustand zumeist in Briefchen bzw. Folien verpackt in den Handel gebracht. Das Anlegen der Kondome bei der Verwendung erfolgt händisch, nachdem die Verpackung geöffnet worden ist. Dabei ergibt sich eine Vielzahl von Möglichkeiten einer nicht fachgerechten Anwendung, die die Sicherheit der Verhütung gefährdet. Zunächst können die Kondome beim Aufreißen der Verpackung mechanisch durch Fingernägel oder dergleichen geschädigt werden. Weiters ist dem aufgerolltem Kondom nicht unmittelbar ersichtlich, in welcher Richtung dieses abzurollen ist. Bei dem Versuch des Abrollens in falscher Richtung besteht wiederum Gefahr einer Beschädigung. Weiters ist die Schutzwirkung beeinträchtigt, da sich eine etwaige spermizide Beschichtung nur auf einer Seite des Kondoms befindet. Auch wird dadurch das Risiko geschaffen, dass Samen auf die Außenseite des Kondoms gelangt, was die beabsichtigte Wirkung offensichtlich zerstört. Darüber hinaus stellt das Abrollen des Kondoms selbst einen für viele Anwender unangenehmen oder unerwünschten Vorgang dar.
Aus der WO 93/21873 ist ein Gerät zum Applizieren von Kondomen bekannt, das aus einem ringförmigen Rahmen mit wannenförmigem Querschnitt besteht, auf dem ein aufgerolltes Kondom aufgelegt werden kann. Wenn nun der Ring mit aufgelegtem Kondom über den Penis geführt wird, so rollt sich das Kondom dabei ab. Nachteilig bei dieser Ausführung ist, das einerseits der Ring einen relativ großen Durchmesser aufweisen muss, um über den Penis geführt werden zu können, da es sich um einen wesentlichen starren Bauteil handelt. Das eingespannte Kondom wird dadurch sehr stark gedehnt, und es besteht die Gefahr einer Beschädigung durch diese Dehnung. Andererseits ist die Führung des Kondoms durch diese Vorrichtung relativ schlecht, und es besteht die Gefahr, dass es vorzeitig herausspringt.
Weiters ist aus der DE 42 41 441 A ein Applikator für Präservative bekannt, bei dem das Präservativ im wesentlichen mit Unterdruck abgewickelt wird. Es hat sich herausgestellt, das solche Vorrichtungen in der Praxis nicht zuverlässig funktionsfähig sind. Weiters lässt sich die Erzeugung des Unterdruckes auf der einen Seite des Kondoms durch bewegliche Zylinder, oder auch auf andere Art (etwa durch eine Pumpe) und das Anbringen am Penis auf der anderen Seite des Kondoms nicht exakt koordinieren.
Gemäß einem älteren Vorschlag des Patentanmelders ist vorgesehen, dass ein Kondom an einem Ring durch elastische oder gelenkige Halte- und Abrollvorrichtungen aufgespannt wird, an denen Rollen paarweise gegenüberliegend angeordnet sind. Die Herstellung der Rollen ist relativ aufwendig, so dass der Bedarf nach einer kostengünstigen und einfach herzustellenden Vorrichtung besteht.
Die FR 2 727 858 A beschreibt eine Vorrichtung zum Applizieren eines Kondoms, bei der das Kondom in Halteklauen eingespannt ist. Das Einbringen des Kondoms in die Vorrichtung ist jedoch nur händisch möglich, was eine Massenfertigung unwirtschaftlich macht.
Aufgabe der vorliegenden Erfindung ist es, einen Applikatorring für Kondome zu schaffen, bei dem die obigen Nachteile nicht auftreten und der einen sicheren, zuverlässigen und angenehmen Vorgang des Anbringens von Kondomen ermöglicht. Eine weitere Aufgabe der Erfindung ist das Schaffen einer Vorrichtung, um das Applizieren von Kondomen mit Hilfe eines solchen Applikatorringes weiter zu vereinfachen.
Erfindungsgemäß wird diese Aufgabe dadurch gelöst, dass die Halteklauen Mittel aufweisen, um eine Stellung zum Einlegen des Kondoms zu erreichen, und dass sie in die das Kondom umschließende Stellung vorgespannt sind. Durch die erfindungsgemäße Ausbildung wird erreicht, dass ein maschinelles Einlegen des Kondoms möglich ist, was den Herstellungsvorgang erleichtert. Weiters wird das Kondom in dem Ring elastisch und nachgiebig gehalten, so dass das Aufbringen des Kondoms für den Anwender keinen unangenehmen Vorgang darstellt. Dabei ist ein Gelenk vorgesehen, durch das die Verbindungsstangen schwenkbar sind, und ein Gelenk, durch das die Verschlussteile beweglich sind.
Wesentlich an der vorliegenden Erfindung ist, dass das Kondom durch Klauen gehalten wird, die in gleichmäßigen Abständen am Umfang des Ringes verteilt angeordnet sind. Gegebenenfalls können auch mehr als drei, beispielsweise fünf, Klauen vorgesehen sein. Mindestens jedoch sind zwei Klauen erforderlich. Dadurch wird die Öffnung des Kondoms sicher in einer für das Einführen des Penis geeigneten Stellung gehalten. Der Gesamtaufbau des Applikatorrings ist so nachgiebig, dass eine Anpassung an die verschiedensten Einsatzbedingungen automatisch und in sanfter Weise erfolgt.
Vorzugsweise ist vorgesehen, dass die Halteklauen eine zylindrische Durchgangsöffnung aufweisen, deren Durchmesser größer ist als der Durchmesser des Wulst des Kondoms in aufgerolltem Zustand und dass die Durchgangsöffnung in Längsrichtung einen Schlitz aufweist, um das Kondom abrollen zu können, welcher Schlitz in seiner Breite kleiner ist, als die Dicke des Wulstes in aufgerolltem Zustand. Dadurch wird erreicht, dass das Kondom in aufgerolltem Zustand sicher in der Durchgangsöffnung gehalten wird, und erst dann durch den Schlitz freigegeben werden kann, wenn der Wulst durch das Abrollen klein geworden ist. Mit zunehmender Abrollung des Kondoms wird die Haltekraft entsprechend kleiner. Wenn der Applikatorring bei nur teilweise abgerolltem Kondom abgezogen werden soll, so kann dies durch einen kleinen Ruck erfolgen. Durch die schwenkbare Befestigung der Halteklaue wird das Abziehen weiter erleichtert.
In einer ersten bevorzugten Ausführungsvariante der Erfindung ist vorgesehen, dass die Klauen jeweils aus einem im Querschnitt hakenförmigen Bauteil bestehen, sowie aus einem Abdeckteil, der an dem hakenförmigen Teil befestigbar ist. Vorzugsweise ist dabei der Abdeckteil mit einem Schnappverschluss am hakenförmigen Teil befestigt. Bei dieser Ausführungsvariante kann das Kondom bei der Herstellung leicht in den Applikatorring eingelegt werden, solange die Abdeckteil noch nicht angebracht ist. Durch Aufklippen oder anderweitige Befestigung des Abdeckteils wird dann das Kondom in der Durchgangsöffnung eingeschlossen.
Alternativ dazu kann vorgesehen sein, dass die Halteklauen einstückig aus einem elastischen Material hergestellt sind. Bei dieser Ausführungsvariante ist die Anzahl der Einzelteile des Applikatorrings verringert und dadurch ist eine weitere Vereinfachung des Herstellungsvorganges möglich. Bei der Montage kann das Kondom durch einen geeigneten Verpackungsautomaten unter Verformung der Klaue in die Durchgangsöffnung eingedrückt werden. So können beispielsweise in den Halteklauen auch Öffnungen vorgesehen sein, um durch das Einführen von Stiften die Klauen zum Einlegen des verpackten Kondoms öffnen zu können. Auf diese Weise kann ein Verpackungsautomat besonders vorteilhaft eingesetzt werden. Bei einer solchen Ausführungsvariante kann insbesondere der gesamte Applikatorring einstückig aus Kunststoff hergestellt werden, wobei die entsprechenden Gelenke durch Stellen geringer Materialstärke gebildet sind. Wichtig ist dabei stets, dass durch das Einführen des Stifts keine Grate entstehen, die eine Verletzung beim Anlegen des Kondoms verursachen können.
Besonders bevorzugt ist es, wenn die Verbindungsstangen zwischen einer ersten und einer zweiten Endlage schwenkbar am Applikatorring angeordnet sind, wobei sie in der ersten Endlage im wesentlichen in der Ebene des Applikatorrings liegen, während sie in der zweiten Endlage im wesentlichen rechtwinkelig zu der Ebene des Applikatorrings liegen, und dass der Schlitz der Halteklauen in die Richtung der Schwenkbewegung der zugehörigen Verbindungsstange orientiert ist. Auf diese Weise kann der Abrollvorgang besonders leicht und reibungsarm erfolgen.

Zur Erfüllung aller gesetzlichen Voraussetzungen und zur Erhöhung der Sicherheit ist es zweckmäßig, die Ringe zu verpacken - genau in der Art der einzeln verpackten Kondome. Um das Auspacken der Kondome zu erleichtern, kann der Ring an seinem äußeren Umfang eine Schneide aufweisen, die unter Druckbeanspruchung die Verpackung aufschneidet.
Die vorliegende Erfindung betrifft weiters eine Vorrichtung zum Auspacken von Applikatorringen, wie sie oben beschrieben worden sind, mit einem Gehäuse, in dem ein Schieber verschiebbar geführt ist. Der Schieber nimmt dabei den Ring auf, so dass sich dieser kaum bewegen kann. Durch einen Schlitz kann die Folie, mit der der Ring verpackt ist, abgezogen werden, ohne dass sich der Ring wesentlich bewegt.
Alternativ dazu kann eine "Kondompistole" verwendet werden, wie sie in der WO 98/01095 beschrieben ist.
In der Folge wird die Erfindung anhand der in den Figuren dargestellten Ausführungsbeispielen näher erläutert. Es zeigen die
- Fig. 1: eine Draufsicht auf einen erfindungsgemäßen Applikatorring,
- Fig. 2: einen Schnitt nach Linie II-II in vergrößertem Maßstab,
- Fig. 3: eine Ansicht des Ringes,
- Fig. 4: einen Schnitt nach Linie IV-IV in Fig. 3,
- Fig. 5: eine Ansicht einer Verbindungsstange in einem vergrößerten Maßstab,
- Fig. 6 und 7: eine Vorrichtung zum Auspacken eines Applikatorrings,
- Fig. 8 und 9: eine weitere Ausführungsvariante eines Applikatorrings in einem Schnitt bzw. einer Draufsicht, und die
- Fg. 10: eine Ausführungsvariante einer Vorrichtung zum Auspacken eines Applikatorrings.

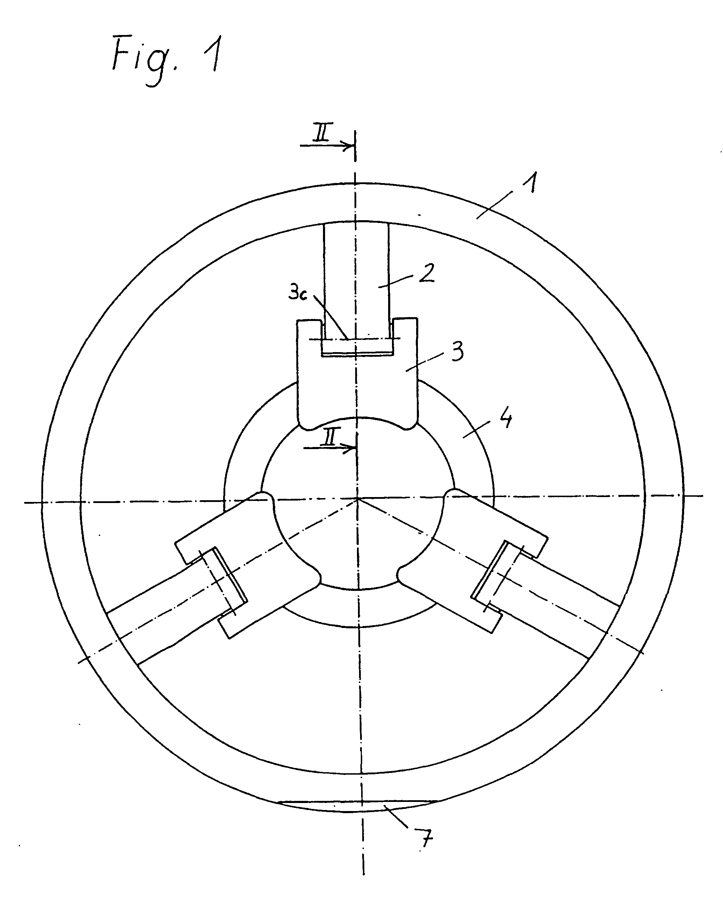

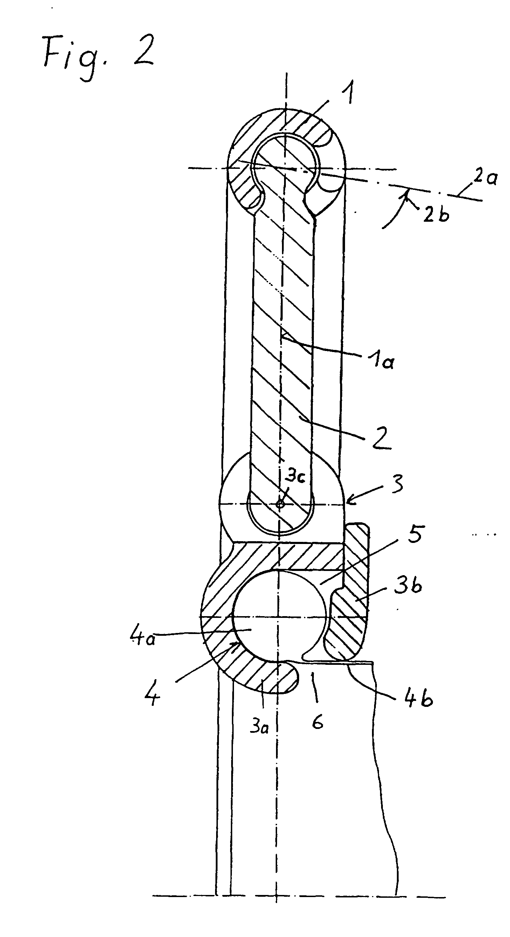

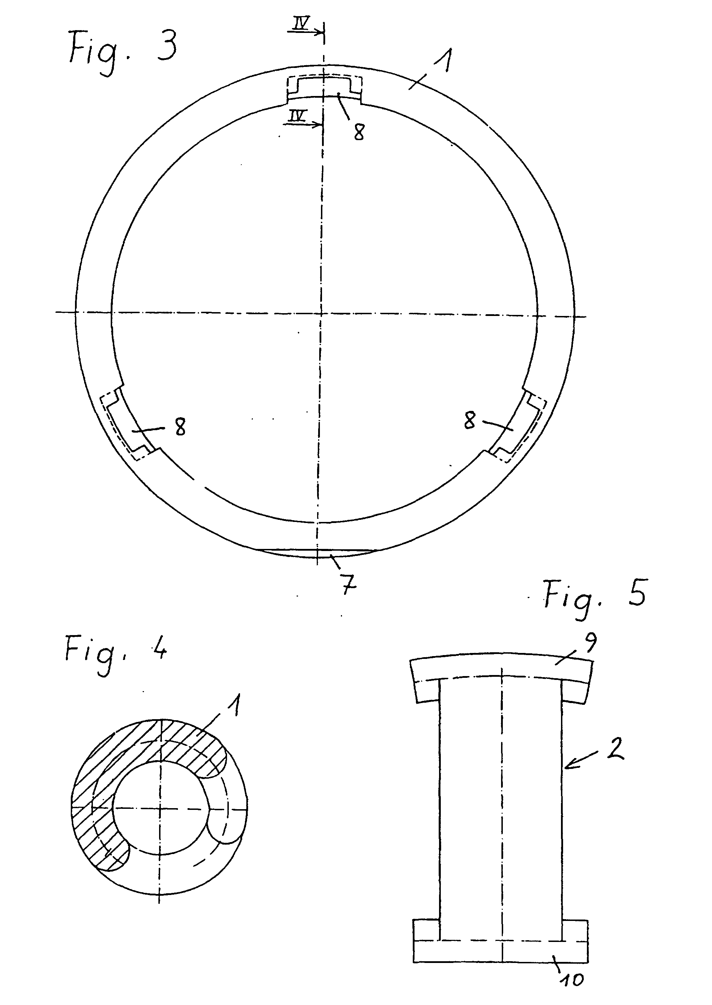

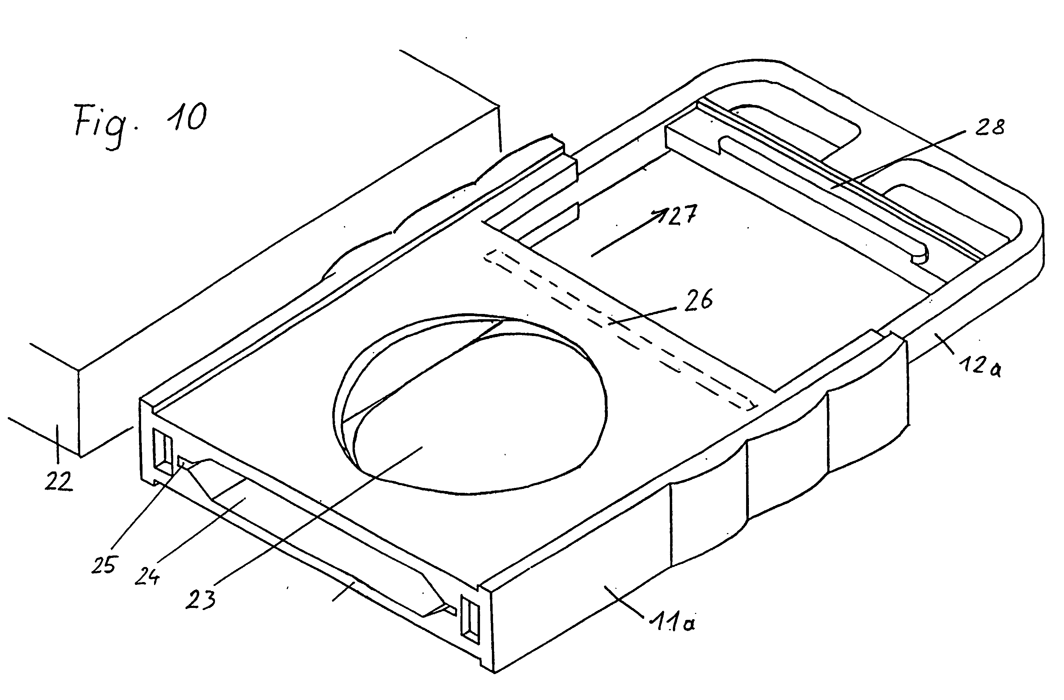

Der Applikatorring von Fig. 1 besteht aus einem Ring 1, an dem in gleichmäßigen Winkelabständen drei Verbindungsstangen 2 gelenkig befestigt sind. Am inneren Ende jeder Verbindungsstange 2 ist eine Halteklaue 3 vorgesehen, die ein Kondom 4 hält. Die Halteklaue 3 ist um eine Achse 3c schwenkbar an der jeweiligen Verbindungsstange 2 angebracht. Aus der Fig. 2 ist ersichtlich, dass die Halteklauen 3 aus einem hakenförmigen Bauteil 3a und einem Abdeckteil 3b bestehen, der durch einen Schnappverschluss am Bauteil 3a befestigt ist. Der hakenförmige Teil 3a und der Abdeckteil 3b schließen eine Durchgangsöffnung 5 ein, wobei zwischen dem hakenförmigen Teil 3a und dem Abdeckteil 3b ein Schlitz 6 ausgebildet ist. Der Wulst 4a des Kondoms 4 ist in der Durchgangsöffnung 5 aufgenommen und der sackförmige Abschnitt 4b des Kondoms 4 wird über den Schlitz 6 nach außen geführt. Weiters ist aus der Fig. 2 ersichtlich, dass die Verbindungsstange 2 zwischen der ersten Endlage, wie sie in dieser Fig. dargestellt ist, und einer durch die Mittellinie 2a angedeuteten Endlage in der Richtung des Pfeils 2b schwenkbar angeordnet ist. Es ist ersichtlich, dass in der zweiten Endlage die Verbindungsstangen 2 nahezu senkrecht auf die Ebene 1a des Applikatorrings 1 stehen.
An einer Stelle seines äußeren Umfangs besitzt der Ring 1 eine Schneide 7, die die Verpackung aufschneidet, wenn eine Zugbeanspruchung in dieser Richtung ausgeübt wird. Die Schneide 7 kann wie in der dargestellten Ausführungsvariante an einer Stelle des äußeren Umfangs ausgebildet sein, sie kann jedoch auch umlaufend ausgeführt sein.
Aus der Fig. 3 ist ersichtlich, dass in dem Ring 1 an drei gleichmäßig am Umfang verteilten Stellen Lager 8 ausgebildet sind, um die Verbindungsstangen 2 gelenkig zu halten. Die Ausbildung der Lager 8 ist in der Fig. 4 im Schnitt dargestellt. Die Verbindungsstangen 2 besitzen einen gekrümmten Zapfen 9 und einen geraden Zapfen 10. Der gekrümmte Zapfen 9 ist dazu ausgebildet, in die Lager 8 des Rings 1 einzurasten. Der gerade Zapfen 10 hält die jeweilige Halteklaue 3.

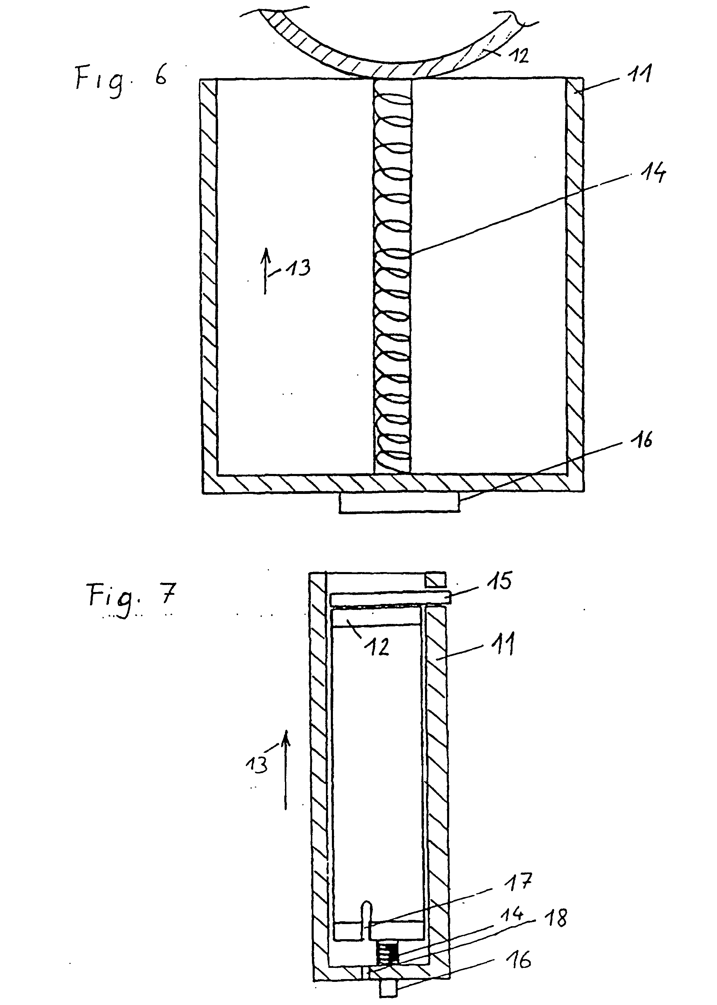

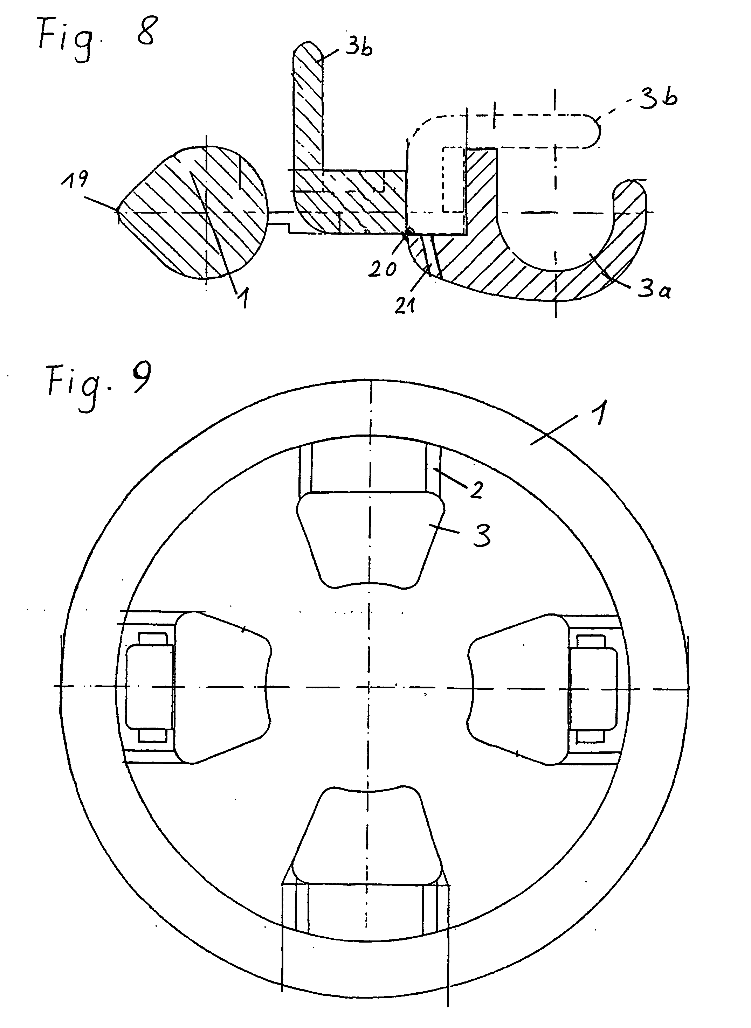

In den Fig. 6 und 7 ist eine Ausführungsvariante der Vorrichtung zum Auspacken von Applikatorringen dargestellt. Die erfindungsgemäße Vorrichtung besteht aus einem Gehäuse 11, in dem ein Schieber 12 in der Richtung des Pfeils 13 verschiebbar gelagert ist. In der Fig. 6 ist der Schieber 12 ist in ausgefahrenem Zustand dargestellt, während in der Fig. 7 der Schieber 12 vollständig innerhalb des Gehäuses 11 angeordnet ist. Eine Feder 14 spannt den Schieber 12 nach oben hin vor. In der in der Fig. 7 dargestellten Stellung wird der Schieber 12 von einem Sperrstift 15 in der dargestellten Stellung gehalten.
Um die Verpackung des nicht dargestellten Applikatorrings entfernen zu können, ist im unteren Bereich des Schiebers 12 eine erste Öffnung 17 vorgesehen, und fluchtend zu dieser ersten Öffnung 17 ist im Gehäuse 11 eine zweite Öffnung 18 vorgesehen. Die Öffnungen 17, 18 können nach oben hin konisch ausgeführt sein, so dass eine entsprechende Lasche der Verpackung des Applikatorrings durch diese Öffnungen 17, 18 nach unten hin herausgeführt werden kann. Die Lasche kann umgebogen und am Knopf 16 befestigt werden. Wenn nun der Sperrstift 15 entfernt wird, drückt die Feder 14 den Schieber 12 samt den eingespannten Applikatorring nach oben, wobei jedoch die Verpackung unten festgehalten wird. Die im oberen Bereich des Applikatorrings angeordnete Schneide 7 schneidet die Verpackung von innen auf, so dass der Schieber 12 mit dem nunmehr unverpackten Applikatorring die in der Fig. 6 dargestellte Stellung erreichen kann. Die Verpackung verbleibt dabei im Inneren des Gehäuses 11. Sie kann in der Folge durch die Schlitze 17, 18 vollständig herausgezogen und entsorgt werden.
Die in der Fig. 8 und 9 dargestellte Ausführungsvariante besitzt für jede Halteklaue 3 zwei elastische Verbindungsstangen 2, die am Ring 1 angebracht sind. Der Ring 1 besitzt im Gegensatz zu der oben beschriebenen Ausführungsvariante an seinem äußeren Umfang eine Schneide 19, um die nicht dargestellte Verpackung des Applikatorrings durch einen entsprechenden Druck zu öffnen.
Die Halteklaue 3 besteht aus einem hakenförmigen Teil 3a und einem Abdeckteil 3b, der durchgezogen in der Stellung zum Einlegen des Kondoms dargestellt ist, während die Stellung nach dem Einlegen des Kondoms in unterbrochenen Linien dargestellt ist. Der Abdeckteil 3b ist an einer Materialverdünnung 20 gelenkig am hakenförmigen Teil 3a gelagert, und wird durch die elastischen Kräfte des Materials in der geschlossenen Stellung (unterbrochene Linien) gehalten. Im unteren Bereich des hakenförmigen Teils 3a ist eine Bohrung 21 vorgesehen, um während des Einlegevorgangs einen nicht dargestellten Stift einführen zu können, um den Abdeckteil 3b aufzudrücken. Das Kondom kann dann von oben leicht eingelegt werden.
Bei der Vorrichtung von Fig. 10 ist in einem Gehäuse 11a ein Schieber 12a gleitbar angeordnet. Ein nur teilweise dargestellter Deckel, der mit dem Gehäuse verbunden sein kann, dient zum Verschließen der Vorrichtung vor dem Gebrauch. In dem Gehäuse 11a ist eine etwa kreisrunde Ausnehmung 23 vorgesehen, durch die der Penis beim Aufbringen des Kondoms hindurchgesteckt werden kann. Der oben beschriebene Applikatorring wird in einem Raum 24 gehalten, der seitlich Nuten 25 für die überstehenden Enden der Verpackung aufweist. Der Raum 24 ist in Öffnungsrichtung des Schiebers (Pfeil 27) mit Ausnahme eines Spalts 26 verschlossen, durch welchen Spalt 26 eine Lasche der Verpackung des Applikatorrings hindurchtreten kann. Die hindurchtretende Lasche wird an einer Haltefläche 28 des Schiebers 12a festgehalten und durch das Herausziehen des Schiebers 12a in Richtung des Pfeils 27 abgezogen. Die Schneide 19 des Applikatorrings schneidet dabei die Verpackung an der gegenüberliegenden Seite auf. Nach Entfernen der Verpackung befindet sich die Vorrichtung in gebrauchsfertigem Zustand.
Die vorliegende Erfindung ermöglicht es, Kondome so verpackt auf den Markt zu bringen, dass bei der Anwendung die Möglichkeit von Fehlbedienungen praktisch ausgeschlossen ist. Die Sicherheit bei der Verhütung wird dadurch erheblich gesteigert.

## Patentansprüche

1. Applikatorring für Kondome mit einem Ring (1), der das Kondom (4) aufgespannt hält, wobei an dem Ring (1) mindestens drei Verbindungsstangen (2) gelenkig angebracht sind, an deren Enden jeweils eine elastische Halteklaue (3) schwenkbar befestigt ist, die aus zwei Verschlussteilen besteht, die einstückig an der zugehörigen Verbindungsstange angeformt sind, und die dazu ausgebildet ist, den Wulst (4a) des Kondoms (4) zu umfassen, **dadurch gekennzeichnet, dass** die Halteklauen (3) Mittel aufweisen, um eine Stellung zum Einlegen des Kondoms zu erreichen, und dass sie in die das Kondom umschließende Stellung vorgespannt sind.

2. Applikatorring nach Anspruch 1, **dadurch gekennzeichnet, dass** die Halteklauen (3) eine zylindrische Durchgangsöffnung aufweisen, deren Durchmesser größer ist als der Durchmesser des Wulstes (4a) des Kondoms (4) in aufgerolltem Zustand und dass die Durchgangsöffnung in Längsrichtung einen Schlitz (6) aufweist, um das Kondom (4) abrollen zu können, welcher Schlitz (6) in seiner Breite kleiner ist, als die Dicke des Wulstes (4a) des Kondoms (4) in aufgerolltem Zustand.

3. Applikatorring nach einem der Ansprüche 1 oder 2, **dadurch gekennzeichnet, dass** die Halteklauen (3) jeweils aus einem im Querschnitt hakenförmigen Bauteil (3a) bestehen, sowie aus einem Abdeckteil (3b), der an dem hakenförmigen Teil befestigbar ist.

4. Applikatorring nach Anspruch 3, **dadurch gekennzeichnet, dass** der Abdeckteil (3b) mit einem Schnappverschluss am hakenförmigen Teil (3a) befestigt ist.

5. Applikatorring nach einem der Ansprüche 1 bis 4, **dadurch gekennzeichnet, dass** am äußeren Umfang des Ringes (1) eine Schneide (7) zum Öffnen einer Verpackung ausgebildet ist.

6. Applikatorring nach einem der Ansprüche 1 bis 5, **dadurch gekennzeichnet, dass** die Verbindungsstangen (2) zwischen einer ersten und einer zweiten Endlage schwenkbar am Applikatorring (1) angeordnet sind, wobei sie in der ersten Endlage im wesentlichen in der Ebene des Applikatorrings (1) liegen, während sie in der zweiten Endlage im wesentlichen rechtwinkelig zu der Ebene des Applikatorrings (1) liegen, und dass der Schlitz (2) der Halteklauen (3) in die Richtung der Schwenkbewegung der zugehörigen Verbindungsstange (2) orientiert ist.

7. Vorrichtung zum Auspacken von Applikatorringen nach einem der Ansprüche 1 bis 6, mit einem Gehäuse (11a), in dem ein Schieber (12a) gleitbar geführt ist, **dadurch gekennzeichnet, dass** das Gehäuse (11a) zur Aufnahme des verpackten Applikatorrings (1) ausgebildet ist und dass der Schieber (12a) eine Haltefläche zum Abziehen der Verpackung aufweist.

8. Vorrichtung nach Anspruch 7, **dadurch gekennzeichnet, dass** das Gehäuse (11a) einen Raum (24) zur Aufnahme des verpackten Applikatorrings (1) besitzt, der seitlich Nuten (25) für die überstehenden Enden der Verpackung aufweist.

9. Vorrichtung zum Auspacken von Applikatorringen nach einem der Ansprüche 1 bis 6, mit einem Gehäuse (11), in dem ein Schieber (12) gleitbar geführt ist, **dadurch gekennzeichnet, dass** der Schieber (12) zur Aufnahme des verpackten Applikatorrings (1) ausgebildet ist und dass das Gehäuse (11) eine Haltefläche zum Abziehen der Verpackung aufweist.

10. Vorrichtung nach Anspruch 9, **dadurch gekennzeichnet, dass** zur Bewegung des Schiebers (12) ein Ausstoßglied (14) vorgesehen ist, dessen Bewegungsrichtung im wesentlichen rechtwinkelig auf die Achse des Applikatorrings (1) angeordnet ist.

11. Vorrichtung nach Anspruch 10, **dadurch gekennzeichnet, dass** das Ausstoßglied (14) mechanisch, hydraulisch oder pneumatisch angetrieben ist.

12. Vorrichtung nach einem der Ansprüche 7 bis 11, **dadurch gekennzeichnet, dass** der Schieber (12; 12a) und/oder das Gehäuse (11; 11a) einen Schlitz (17, 18, 26) zum Abziehen der Verpackung aufweist.

## Claims

1. Applicator ring for condoms with a ring (1) that keeps the condom (4) tense, wherein at least three connecting rods (2) are articulated on the ring (1), an elastic retaining claw (3) consisting of two locking parts formed integral with the corresponding connecting rod being pivotally attached at the end of each rod and being designed to clutch the roll (4a) of the condom (4), **characterized in that** the retaining claws (3) are provided with means serving to achieve a position for inserting the condom and that they are pretensioned in the position encompassing the condom.

2. Applicator ring according to claim 1, **characterized in that** the retaining claws (3) are provided with a cylindrical through hole, the diameter of which being greater than the diameter of the roll (4a) of the condom (4) when it is rolled up, and that the through hole is provided in longitudinal direction with a slot (6) serving to unroll the condom (4), said slot (6) being smaller in width than the thickness of the roll (4a) of the rolled-up condom (4).

3. Applicator ring according to one of the claims 1 or 2, **characterized in that** the retaining claws (3) each consist of a component part (3a) of a hook-shaped cross section and of a covering part (3b) that may be fastened to said hook-shaped part.

4. Applicator ring according to claim 3, **characterized in that** the covering part (3b) is fastened on the hook-shaped part (3a) with a snap.

5. Applicator ring according to one of the claims 1 through 4, **characterized in that** a cutting edge (7) designed to open a packaging is formed on the outer periphery of the ring (1).

6. Applicator ring according to one of the claims 1 through 5, **characterized in that** the connecting rods (2) are pivotally arranged on the applicator ring (1) between a first and a second end position, wherein, in the first end position, they are level with the plane of the applicator ring (1), whereas in the second end position they are essentially at right angles to the plane of the applicator ring (1) and that the slot (2) of the retaining claws (3) is oriented in the direction of the pivotal movement of the corresponding connecting rod (2).

7. Device for unpacking applicator rings according to one of the claims 1 through 6 with a housing (11a), in which a tab (12a) is slidably guided, **characterized in that** the housing (11a) is designed to receive the packaged applicator ring (1) and that the tab (12a) is provided with a retaining surface for pulling off the packaging.

8. Device according to claim 7, **characterized in that** the housing (11a) holds a space (24) designed to receive the packaged applicator ring (1) and being provided on its sides with grooves (25) for the proud ends of the packaging.

9. Device for unpacking applicator rings according to one of the claims 1 through 6 with a housing (11), in which a tab (12) is slidably guided, **characterized in that** the tab (12) is designed to receive the packaged applicator ring (1) and that the housing (11) is provided with a retaining surface for pulling off the packaging.

10. Device according to claim 9, **characterized in that** an expelling member (14) is provided for moving the tab (12), the moving direction of said expelling member being essentially arranged at right angles to the axis of the applicator ring (1).

11. Device according to claim 10, **characterized in that** the drive of the expelling member (14) is mechanical, hydraulic or pneumatic.

12. Device according to one of the claims 7 to 11, **characterized in that** the tab (12; 12a) and/or the housing (11; 11a) are provided with a slot (17, 18, 26) serving to pull off the packaging.

## Revendications

1. Anneau applicateur pour préservatifs avec un anneau (1) maintenant le préservatif (4) tendu, au moins trois baguettes de liaison (2) étant articulées à l'anneau (1), une griffe de retenue (3) élastique constituée de deux éléments de fermeture venus de moulage d'une seule pièce avec la baguette de liaison associée étant fixée pivotante à chaque extrémité desdites baguettes et étant destinée à enserrer le bourrelet (4a) du préservatif (4), **caractérisé en ce que** les griffes de retenue (3) comportent des moyens servant à atteindre une position permettant d'insérer le préservatif et qu'elles sont précontraintes dans la position enserrant le préservatif.

2. Anneau applicateur selon la revendication 1, **caractérisé en ce que** les griffes de retenue (3) comportent un trou de passage cylindrique dont le diamètre est supérieur au diamètre du bourrelet (4a) du préservatif (4) enroulé et que, dans la direction longitudinale, le trou de passage comporte une fente (6) permettant de dérouler le préservatif (4), la largeur de ladite fente (6) étant inférieure à l'épaisseur du bourrelet (4a) du préservatif (4) enroulé.

3. Anneau applicateur selon l'une quelconque des revendications 1 ou 2, **caractérisé en ce que** les griffes de retenue (3) sont constituées chacune d'un élément constitutif (3a) de section en forme de crochet ainsi que d'un élément de recouvrement (3b) apte à être fixé à l'élément en forme de crochet.

4. Anneau applicateur selon la revendication 3, **caractérisé en ce que** l'élément de recouvrement (3b) est fixé à l'élément en forme de crochet (3a) par une fermeture à ressort.

5. Anneau applicateur selon l'une quelconque des revendications 1 à 4, **caractérisé en ce qu'**une arête coupante (7) destinée à ouvrir un emballage est réalisée sur la circonférence extérieure de l'anneau (1).

6. Anneau applicateur selon l'une quelconque des revendications 1 à 5, **caractérisé en ce que** les baguettes de liaison (2) sont disposées sur l'anneau applicateur (1) de manière à pouvoir pivoter entre une première et une deuxième position extrême, lesdites baguettes se situant sensiblement dans le plan de l'anneau applicateur (1) dans la première position extrême alors que dans la deuxième position extrême elles sont sensiblement perpendiculaires au plan de l'anneau applicateur (1) et que la fente (2) des griffes de retenue (3) est orientée selon la direction de pivotement de la baguette de liaison (2) correspondante.

7. Dispositif destiné au déballage d'anneaux applicateurs selon l'une quelconque des revendications 1 à 6, avec un boîtier (11a) dans lequel est guidé à coulissement un coulisseau (12a), **caractérisé en ce que** le boîtier (11a) est conformé pour recevoir l'anneau applicateur (1) dans son emballage et que le coulisseau (12a) comporte une surface de retenue servant à retirer l'emballage.

8. Dispositif selon la revendication 7, **caractérisé en ce que** le boîtier (11a) possède une enceinte (24) destinée à recevoir l'anneau applicateur (1) emballé et comportant sur ses côtés des rainures (25) pour les extrémités saillantes de l'emballage.

9. Dispositif destiné au déballage d'anneaux applicateurs selon l'une quelconque des revendications 1 à 6, avec un boîtier (11) dans lequel est guidé à coulissement un coulisseau (12), **caractérisé en ce que** le coulisseau (12) est conformé pour recevoir l'anneau applicateur (1) emballé et que le boîtier (11) comporte une surface de retenue servant à retirer l'emballage.

10. Dispositif selon la revendication 9, **caractérisé en ce qu'**un membre éjecteur (14) servant à déplacer le coulisseau (12) est prévu, lequel membre éjecteur se déplace selon une direction sensiblement perpendiculaire à l'axe de l'anneau applicateur (1).

11. Dispositif selon la revendication 10, **caractérisé en ce que** le membre éjecteur (14) est à entraînement mécanique, hydraulique ou pneumatique.

12. Dispositif selon l'une quelconque des revendications 7 à 11, **caractérisé en ce que** le coulisseau (12 ; 12a) et/ou le boîtier (11 ; 11a) comportent une fente (17, 18, 26) servant à retirer l'emballage.
